(19) Europäisches Patentamt / European Patent Office / Office européen des brevets

(11) **EP 3 741 341 A1**

(12) **EUROPEAN PATENT APPLICATION**

(43) Date of publication:
**25.11.2020 Bulletin 2020/48**

(51) Int Cl.:
**A61F 13/514** *(2006.01)*  **A01K 23/00** *(2006.01)*
**A61B 46/00** *(2016.01)*

(21) Application number: **20175178.1**

(22) Date of filing: **18.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.05.2019  JP 2019094871**
**11.11.2019  JP 2019203979**

(71) Applicant: **Unicharm Corporation**
**Shikokuchuo-shi, Ehime 799-0111 (JP)**

(72) Inventor: **KOMATSUBARA, Daisuke**
**Kanonji-shi, Kagawa 769-1602 (JP)**

(74) Representative: **Staeger & Sperling**
**Partnerschaftsgesellschaft mbB**
**Sonnenstraße 19**
**80331 München (DE)**

(54) **DISPOSABLE DIAPER FOR PET AND METHOD FOR MANUFACTURING DISPOSABLE DIAPER FOR PET**

(57)    A disposable diaper for pet (1) includes a waistline direction (W) disposed along a waistline direction of a pet, a cross direction (Z) orthogonal to the waistline direction, a main body portion (2), and a tail hole (70) through which a tail of pet can be inserted. The main body portion (2) has a topsheet (10), a backsheet (20), and an absorbent core (30) arranged between the topsheet and the backsheet. The disposable diaper for pet has a tail mark portion (50) that indicates a position of the tail hole.

FIG. 1

# Description

TECHNICAL FIELD

The present invention relates to a disposable diaper for pet placed on a pet such as dog and cat.

[BACKGROUND ART]

Patent Literature 1 discloses a disposable diaper for pet. The disposable diaper for pet disclosed in Patent Literature 1 has a belly pad to be placed on the ventral side of a pet, a back pad to be placed on the dorsal side of pet, a tail hole allowed for insertion of a tail through the back pad, and a fastener tape arranged to the belly pad. When the disposable diaper for pet disclosed in Patent Literature 1 is placed, the belly pad is placed on the ventral side of pet and the back pad is placed on the dorsal side of the pet, while the tail is inserted through the tail hole, and the fastener tape is attached on the back pad (see Fig. 4 of Patent Literature 1).

[CITATION LIST]

[PATENT LITERATURE]

[Patent Literature 1] JP 2003-210062 A

[SUMMARY OF INVENTION]

The disposable diaper for pet described in Patent Literature 1 has, however, suffered from a problem below.
The tail hole is small relative to the entire range of the main body portion, and some user even has placed the disposable diaper, while being unaware of the tail hole. As a result of being unaware of the presence of the tail hole, the user would have misjudged the direction or position of placement of the main body portion, and would have failed in placement in the suitable position.

It is therefore an object of the present invention to provide a disposable diaper for pet having the tail hole, which can be placed in the suitable position.

A disposable diaper for pet according to one embodiment includes: a waistline direction disposed along a waistline direction of a pet; a cross direction orthogonal to the waistline direction, and extends in a direction connecting a ventral side and a dorsal side of the pet; a main body portion having a topsheet, a backsheet, and an absorbent core arranged between the topsheet and the backsheet; and a tail hole through which a tail of pet can be inserted. The disposable diaper for pet has a tail mark portion that indicates a position of the tail hole.

A method for manufacturing disposable diaper for pet according to one embodiment includes a sheet feeding step of feeding a continuous sheet having sheet components arrayed in the continuous sheet; a marking step of giving a tail mark portion on the continuous sheet;

and a core laminating step of laminating an absorbent core on the continuous sheet having the tail mark portion. The core laminating step is designed to detect a position of the tail mark portion on the continuous sheet, and to adjust a position of lamination of the absorbent core referring to the position of the tail mark portion.

BRIEF DESCRIPTION OF DRAWINGS

[Fig. 1] Fig. 1 is a plan view in which a disposable diaper according to a first embodiment of the present invention is seen from a skin contact surface side.
[Fig. 2] Fig. 2 is a plan view illustrating the disposable diaper for pet of the first embodiment, when viewed from the non-skin contact side.
[Fig. 3] Fig. 3 is a schematic cross sectional view of the disposable diaper for pet taken along the line A-A in Fig. 1.
[Fig. 4] Fig. 4 is a drawing schematically illustrating a wearing state of the disposable diaper for pet.
[Fig. 5] Fig. 5 are plan views in which a disposable diaper according to second embodiment to fifth embodiment of the present invention are seen from a skin contact surface side.

(1) Outline of Embodiment

According to the present specification and the accompanying drawings, at least the following matters will be disclosed.
A disposable diaper for pet according to one embodiment includes: a waistline direction disposed along a waistline direction of a pet; a cross direction orthogonal to the waistline direction, and extends in a direction connecting a ventral side and a dorsal side of the pet; a main body portion having a topsheet, a backsheet, and an absorbent core arranged between the topsheet and the backsheet; and a tail hole through which a tail of pet can be inserted. The disposable diaper for pet has a tail mark portion that indicates a position of the tail hole. According to this embodiment, the user can be aware of the presence of the tail hole with the aid of the tail mark portion, and can insert the tail through the tail hole to place the disposable diaper. The placement of the disposable diaper with the tail inserted through the tail hole can improve the fitting quality of the disposable diaper.

According to a preferred embodiment, a minimum distance, in plan view of the disposable diaper for pet, between the tail mark portion and the tail hole is equal to or smaller than a maximum size of the tail hole. According to this embodiment, the user will become easy to visually recognize both of the tail mark portion and the tail hole at the same time, and will become more aware of the presence of the tail hole with the aid of the tail mark portion.

According to a preferred embodiment, at least a part of the tail mark portion, in plan view of the disposable diaper for pet, may be arranged in a region that overlaps the tail hole. According to this embodiment, the user will become easy to visually recognize both of the tail mark portion and the tail hole at the same time, and will become more aware of the presence of the tail hole with the aid of the tail mark portion.

According to a preferred embodiment, the tail mark portion indicates a direction of placement of the disposable diaper for pet. According to this embodiment, with the tail mark portion that points the direction of placement of the disposable diaper for pet, the user can recognize a proper direction of placement of the disposable diaper, and can place the disposable diaper in a correct direction. Even in case of placement in the wrong direction, the user can visually recognize the tail mark portion after the placement, and can correct the disposable diaper in the right direction.

According to a preferred embodiment, the tail hole has a through-hole through which the tail of pet can be inserted, and a cut part that can enlarge the through-hole. The tail mark portion indicates a position of the cut part. According to this embodiment, the user can be aware of the presence of the cut part beside the tail hole with the aid of the tail mark portion, and can enlarge the through-hole with the aid of the cut part as necessary. With the through-hole properly formed depending on the size or shape of the tail of pet, the user can place the disposable diaper in a proper place.

According to a preferred embodiment, the cut part has a first cut part, and a second cut part positioned more distant from the through-hole than the first cut part. The tail mark portion has a first tail mark portion that indicates a position of the first cut part, and a second tail mark portion that indicates a position of the second cut part. According to this embodiment, the user can be aware of the presence of the plurality of cut parts, and can adjust the size of the through-hole to a degree suited for the individual pets.

According to a preferred embodiment, the first tail mark portion teaches order of manipulation of the first cut part. The second tail mark portion teaches order of manipulation of the second cut part. According to this embodiment, the user can understand the order of manipulation of the cut parts, and can enlarge stepwise the through-hole by the manipulation, and can thereby adjust the size of the through-hole suited to the individual pets.

According to a preferred embodiment, the tail mark portion teaches a direction of manipulation of the cut part. According to this embodiment, the user can understand the direction of manipulation of the cut part, can enlarge the through-hole by the manipulation, and can thereby adjust the size of the through-hole to a degree suited for the individual pets.

According to a preferred embodiment, the backsheet of the disposable diaper for pet has the tail mark portion, and a decoration part designed differently from the tail mark portion printed on the backsheet. According to this embodiment, the tail mark portion can make the user aware of the presence of the tail hole, while improving the decorativeness of the disposable diaper as a whole. Differently designed decoration part and the tail mark portion can also make the user easily discriminate and recognize them.

According to a preferred embodiment, the tail mark portion may have a design portion representing a wearing target pet. According to this embodiment, the user can recognize the wearing target pet, with the aid of the tail mark portion.

A method for manufacturing disposable diaper for pet according to one embodiment includes a sheet feeding step of feeding a continuous sheet having sheet components arrayed in the continuous sheet; a marking step of giving a tail mark portion on the continuous sheet; and a core laminating step of laminating an absorbent core on the continuous sheet having the tail mark portion. The core laminating step is designed to detect a position of the tail mark portion on the continuous sheet, and to adjust a position of lamination of the absorbent core referring to the position of the tail mark portion. According to this embodiment, the relative relation between the tail mark portion and the absorbent core may be kept constant. Hence, when the disposable diaper is placed referring to the mark portion, placement position of the absorbent core is prevented from being misaligned for the individual disposable diapers. Such suppression of misalignment of the tail mark portion, relative to the disposable diaper as a whole, can improve accuracy of the relative position between the tail mark portion and the tail hole.

(2) Overall Structure of Disposable diaper for pet

A disposable diaper for pet according to an embodiment will be described below by referring to the accompanying drawings. In the drawings, the same or similar parts are indicated by the same or similar reference signs. The drawings are illustrated schematically, and dimensional ratio and other variables differ from those of actual measurements. The actual measurements or the like, therefore, should be determined by referring to the following description. The drawings may include different relationships or ratios of measurements.

In this patent specification, "pet" widely encompasses vertebrate and invertebrate animals, and typically include pet animals such as cat, dog, rabbit and hamster. The disposable diaper in this embodiment is a disposable diaper for dog. In a modified example, the disposable diaper may be the one for cat.

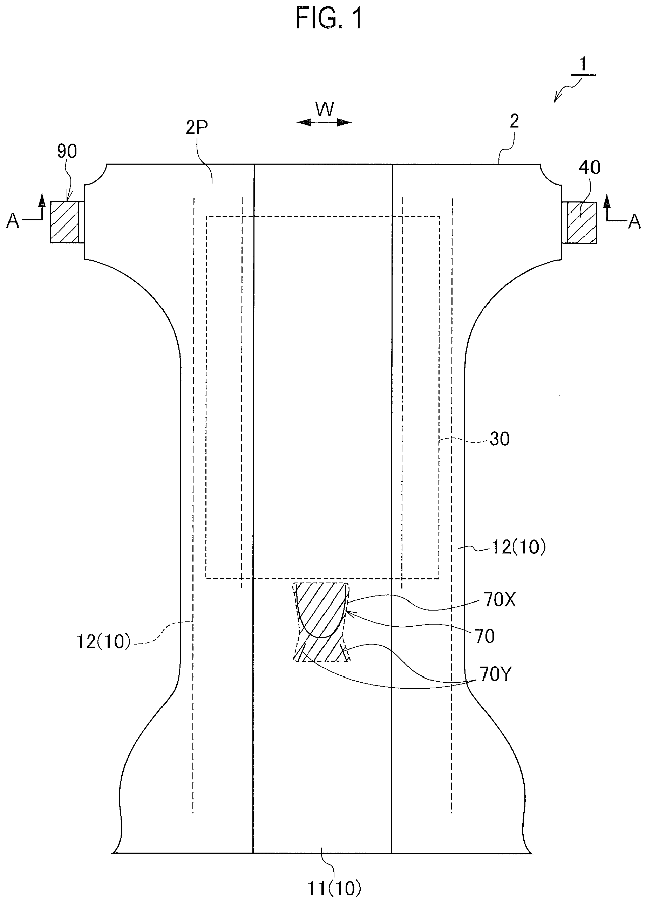

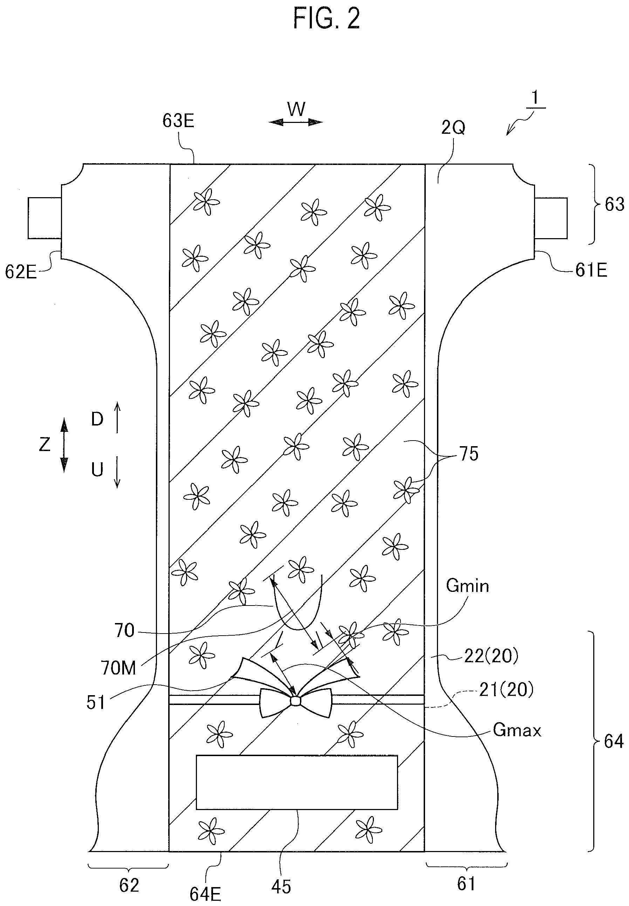

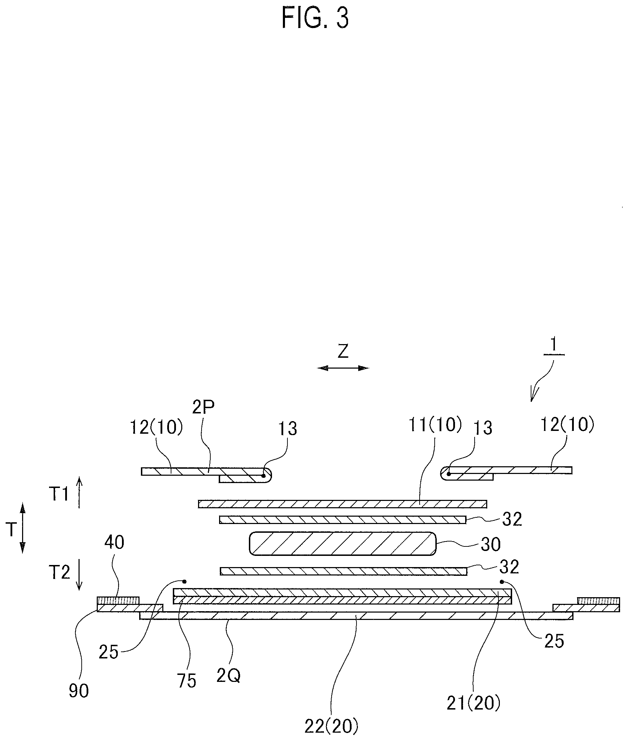

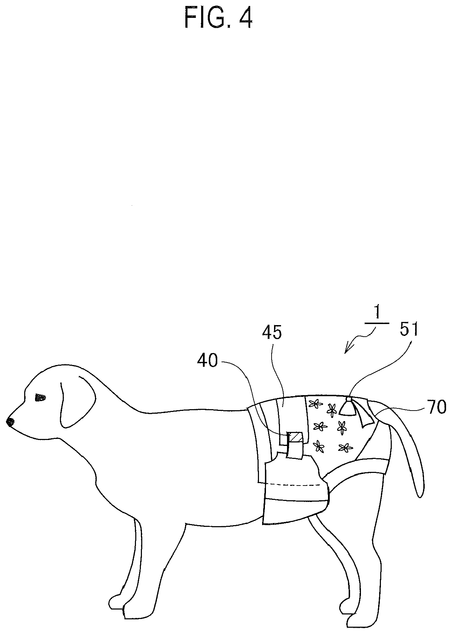

Fig. 1 is a plan view illustrating the disposable diaper for pet of the first embodiment, when viewed from the skin contact side. Fig. 2 is a plan view illustrating the disposable diaper for pet of the first embodiment, when viewed from the non-skin contact side. Fig. 3 is a schematic cross sectional view of the disposable diaper for pet taken along the line A-A in Fig. 1. Figs. 1 and 2 illustrate a disposable diaper for pet 1, extended until any wrinkle will disappear. The individual members, although illustrated in the cross sectional view of Fig. 3 as if they are apart from each other in the thickness direction T for the convenience of explanation, are brought into close contact in the thickness direction T in the actual product. Fig. 4 is a drawing schematically illustrating a wearing state of the disposable diaper for pet.

The disposable diaper for pet has waistline direction W laid in the waistline direction of the pet, cross direction Z laid orthogonal to the waistline direction W, and the thickness direction T laid orthogonal to the waistline direction W and to the cross direction Z. The cross direction Z is laid in the longitudinal direction of the pet, that is, the direction connecting the crotch-side with the far side of the crotch. The thickness direction T is laid towards the top face side T1 faced to the pet in the wearing state, and towards the back face side T2 faced outside in the wearing state. As illustrated in Fig. 4, the disposable diaper for pet of the first embodiment is placed so as to wrap around the waist of the pet, rather than being placed so as to cover the pet from the belly through the crotch to the back.

The disposable diaper for pet 1 has a main body portion 2 and a binding portion 40. The main body portion 2 may have a main-body first end 61 which is one end part in the waistline direction W, a main-body second end 62 which is the other end part in the waistline direction W, a main-body third end 63 which is one end part in the cross direction Z, and a main-body fourth end 64 which is the other end part in the cross direction Z. The main-body first end 61 and the main-body second end 62 have bonded thereto a fastening tape 90 described later. The main-body third end 63 is disposed on the dorsal side of pet in the wearing state. The main-body fourth end 64 is opposed to the main-body third end 63, and is disposed on the ventral side of pet in the wearing state. The end in the context of this invention is defined as a part that occupies a certain range including the edge. The main-body first end 61 is a part that extends inwardly in the waistline direction W, from a first edge 61E which is one edge in the waistline direction W of the main body portion 2. Likewise, the main-body second end 62 is a part that extends inwardly in the waistline direction W, from a second edge 62E which is the other edge in the waistline direction W of the main body portion 2. The main-body first end and the main-body second end 62 may be regions between the edges in the waistline direction W of the main body portion 2 and the edges in the waistline direction W of the absorbent core 30. The main-body third end 63 is a part that extends inwardly in the cross direction Z from a third edge 63E which is one edge in the cross direction Z of the main body portion 2. Likewise, the main-body fourth end 64 is a part that extends inwardly in the cross direction Z, from a fourth edge 64E which is the other edge in the cross direction Z of the main body portion 2. The main-body third end 63 and the main-body fourth end 64 may be regions between the edges in the cross direction Z of the main body portion 2 and the edges in the cross direction Z of the absorbent core 30. The main-body fourth end 64 may be a region that ranges between the edge in the cross direction Z of the main body portion 2 and the edge in the cross direction Z of the absorbent core 30; or may be a region that ranges between the edge in the cross direction Z of the main body portion 2 and a tail hole 70 described later.

The disposable diaper for pet 1 has a main body portion 2 and a binding portion 40. The disposable diaper for pet 1 has a main body portion 2 and a binding portion 40. The main body portion 2 has at least a topsheet 10, a backsheet 20, and an absorbent core 30. The topsheet 10 is arranged to the face to be placed on the pet. The topsheet 10 has liquid permeability that allows body fluid to permeate towards the absorbent core 30. The topsheet 10 may have a center sheet 11 arranged at the center in the cross direction Z so as to cover the absorbent core 30, and side sheets 12 individually covering both side part the cross direction Z of the center sheet 11.

The side sheets 12 may be folded as illustrated in Fig. 3. More specifically, the side sheet 12 is folded back at the inner end of in the cross direction Z of the side sheet 12 towards the back face side T2. A side elastic member 13 while being stretched in the waistline direction W is arranged in between folded parts of the side sheets 12. The side elastic member 13 may form leakproof gathers that rise up towards the pet.

The absorbent core 30 is arranged between the topsheet 10 and the backsheet 20. The absorbent core 30 is made of a stack of pulp or other absorption materials. As illustrated in Fig. 4, the absorbent core 30 may have a core wrap 32 that covers the absorbent core 30. The absorbent core 30 is arranged, in the waistline direction W, inside of both edges of the main body portion 2. That is, the length in the waistline direction W of the absorbent core 30 is shorter than the length in the waistline direction W of the main body portion 2. The absorbent core 30 is arranged at the center in the waistline direction W of the main body portion 2, but is not arranged outside in the waistline direction W of the main body portion 2. The absorbent core 30 may be arranged, in the cross direction Z, inside of both edges of the main body portion 2. That is, the length in the cross direction Z of the absorbent core 30 may be shorter than the length in the cross direction Z of the main body portion 2. The absorbent core 30 may be arranged at the center in the cross direction Z of the main body portion 2, but is not necessarily arranged outside in the cross direction Z of the main body portion 2. The absorbent core and the core wrap may have arranged thereto a printed part. The printed part may form a tail mark portion 51.

The backsheet 20 is arranged on the outer face 2Q of the main body portion 2. The backsheet 20 may have a non-liquid permeable back film 21 and a nonwoven back fabric 22 arranged on the outer side of the back film 21. In a modified example, the backsheet may have a non-liquid permeable back film and a nonwoven back fabric arranged on the back side of the back film 21. The back film 21 may have, on a face thereof on the top face side T1, an indicator that causes a coloring reaction upon contact with body fluid, and a printed part, meanwhile the back film 21 may have, on a face thereof on the back face side T2, a printed part. At least one of the indicator or the printed part may form the tail mark portion 51. That is, the length in the cross direction Z of the back film 21 may be shorter than the length in the cross direction Z of the nonwoven back fabric 22. That is, the nonwoven back fabric 22 may extend on both sides in the cross direction Z out from the back film 21.

The binding portion 40 extends out from both ends in the waistline direction W of the main body portion 2, in the main-body third end 63 which is one end in the cross direction Z of the main body portion 2. In more detail, the binding portion 40 is arranged to the fastening tape 90 that extends, in the main-body third end 63, out from the first edge 61E and the second edge 62E of the main body portion 2 in the waistline direction W. The binding portion 40 is arranged on a face on the top face side T1 of the fastening tape 90. The binding portion 40 may be a mechanical fastener, and is formed so as to be bindable with a target part 45 arranged on an outer face 2Q of the main body portion 2. In a modified example, the main body portion may be formed without the target part, and instead the binding portion 40 may be formed so as to be bindable with the backsheet on the outer face 2Q side of the main body portion 2. In a modified example, the binding portion 40X may extend, in the main-body fourth end 64, out from both ends in the waistline direction W of the main body portion 2X.

The disposable diaper for pet 1 may have formed therein the tail hole 70 through which the tail of pet can be inserted. The tail hole 70 has a through-hole 70X through which the tail of pet can be inserted, and cut parts 70Y that can enlarge the through-hole. When the disposable diaper with the tail hole 70 is placed, the tail of pet may be inserted in the through-hole 70X of the tail hole 70, in the process of covering the hip and back of pet with the other end part in the cross direction Z of the main body portion 2. The through-hole 70X may be a half-arc cut. The cut parts 70Y may have a structure such as perforation, along which the topsheet 10 and the backsheet 20 are tearable, making the size of the through-hole 70X variable depending on the species and growth process of pet. The cut parts in this embodiment are arranged one by one on the left and right sides of the tail hole 70, while placing the center in the waistline direction in between. In another embodiment, two or more cut parts may alternatively be arranged on each of the left and right sides of the tail hole 70 while placing the center in the waistline direction in between, so that the size of the tail hole 70 may be adjustable stepwise.

When the disposable diaper for pet 1 is placed, the main-body third end 63 (end part on the side where the binding portion 40 is arranged) is placed on the belly of pet. In this process, the other end in the cross direction Z of the main body portion 2 is passed between the legs of pet, and left behind the pet. The center in the cross direction Z of the main body portion 2 is then aligned with the excretory opening of pet, with the tail of pet inserted through the tail hole 70 and left on the back face side of the disposable diaper. The hip and back of pet is then covered with the main-body fourth end 64. The binding portion 40 is then pulled up towards the back of the pet, and attached to the outer face of the target part 45 in the main-body fourth end 64 that reside on the dorsal side. Thus, the disposable diaper for pet 1 may be placed as illustrated in Fig 4, so as to cover the belly, back and crotch of pet. That is, the disposable diaper for pet 1 is placed so as to cover the pet from the belly through the crotch to the back of the pet.

Next, the tail mark portion 51 will be explained. The tail mark portion 51 points a position of the tail hole 70. The tail mark portion 51 is made visually recognizable from the backsheet side (outer face side) of the disposable diaper for pet. The backsheet side of the disposable diaper for pet is directed outwards in the wearing state, and is visually recognizable not only in the process of placement of the disposable diaper on a pet, but also after placement of the disposable diaper on the pet. Thus the position of the tail hole 70 may be pointed not only in the process of manipulation for placement, but also after the manipulation for placement. The user will be aware of the presence of the tail hole 70 with the aid of the tail mark portion 51, and can insert the tail through the tail hole 70 to place the disposable diaper. The placement of the disposable diaper with the tail inserted through the tail hole 70 can improve the fitting quality of the disposable diaper. In addition, with the tail inserted through the tail hole 70, the user can properly align the direction of the main body portion for placement in the suitable position.

Now "visually recognizable" in the context of this invention means that a subject with healthy vision in both eyes (20/20 vision or higher) can recognize an object from approximately 30 to 50 cm away, in a bright (approximately 500 to 750 lx) room with daylight illumination (color temperature ranging from 4600 to 5400 K).

The tail mark portion 51 may be arranged in a region around the tail hole 70. The maximum gap Gmax between the tail mark portion 51 and the tail hole 70, in plan view of the disposable diaper for pet, may be equal to or smaller than the maximum size of the tail hole 70. The user will become easy to recognize both of the tail mark portion 51 and the tail hole 70 at the same time, and will become more aware of the presence of the tail hole 70 with the aid of the tail mark portion 51. More preferably, the minimum gap Gmin between the tail mark portion 51 and the tail hole 70 may be equal to or smaller than a maximum size 70M of the tail hole 70. Alternatively, the minimum gap Gmin between the tail hole 70 and the tail mark portion 51 may be 20 mm. The maximum gap between the tail hole 70 and the tail mark portion 51 may preferably be 20 mm. The user will become easier to recognize both of the tail mark portion 51 and the tail hole 70 at the same time, and will become more aware of the presence of the tail hole 70 with the aid of the tail mark portion 51. The maximum size of the tail hole 70 may only be a maximum size in plan view, and may be a size measured in the waistline direction, may be a size measured in the cross direction, or may be a size measured in a direction inclined away from the waistline direction.

The tail mark portion 51 may be arranged in a region that overlaps the tail hole 70. The region that overlaps the tail hole 70 is a region inside of a line connecting the contour of the outline of the tail hole 70, which is hatched in Fig. 1. The user will become easier to recognize both of the tail mark portion 51 and the tail hole 70 at the same time, and will become further aware of the presence of the tail hole 70 with the aid of the tail mark portion 51.

The tail mark portion 51 may point the direction of placement of the disposable diaper for pet. The direction of placement is specifically exemplified by vertical direction and lateral direction in the process of placement. Fig. 2 illustrates an upward direction U which is kept upside in the process of placement, and a downward direction D which is kept downside in the process of placement. In an embodiment where the disposable diaper is placed so as to cover the pet from the belly through the crotch to the back, the user would sometimes fail in correctly discriminating the regions to be placed on the belly and the back, and would accidentally invert the belly side and the back side. Now with the tail mark portion 51 for pointing the direction of placement of the disposable diaper for pet, the direction of placement of the disposable diaper can be pointed during the placement, and thereby the user can place the disposable diaper in a correct direction. Even in case of placement in the wrong direction, the user can visually recognize the tail mark portion 51 after the placement, and can correct the disposable diaper in the right direction.

The tail mark portion 51 that points the direction of placement may point the direction with a design having vertical directionality, or with letter, numeral or symbol having vertical directionality. The disposable diaper placed in the correct direction makes the design or letter directed in the correct direction, making it possible to point the direction of placement of disposable diaper. The tail mark portion 51 in this embodiment has an illustration of ribbon, where the correct direction is given by two loops extending radially from a knot positioned upside, and the trails falling down from the knot positioned downside. The correct direction of placement is thus pointed by the illustration of ribbon.

The backsheet of the disposable diaper for pet may have the tail mark portion 51, and a decoration part 75 designed differently from the tail mark portion 51 printed thereon. The decoration part is a part having a design other than the tail mark portion 51, and may contain not only picture or pattern, but also background color solely represented by color. It now becomes possible to make the user aware of the presence of the tail hole 70 with the aid of the tail mark portion 51, while improving the decorativeness of the disposable diaper as a whole with the aid of the decoration part. Differently designed decoration part and the tail mark portion 51 can also make the user easily discriminate and recognize them. Difference of designs between the decoration part and the tail mark portion 51 may be given by difference in color, shape or pattern.

Color difference ΔE between the decoration part and the tail mark portion 51 may be 3.0 or larger. The color difference ΔE between the decoration part and the tail mark portion 51 may be determined by measuring colors at two target points (two regions) of measurement using a commercially available colorimeter, and by comparing results quantified in CIE 1976 (L*a*b*) color space specified in JIS Z 8729. More specifically, letting difference of L* value between two target points of measurement be ΔL*, difference of a* be Δa*, and difference of b* be Δb*, now the color difference ΔE is given by the equation below:

$$\Delta E = [(\Delta L^*)^2 + (\Delta a^*)^2 + (\Delta b^*)^2]^{1/2}.$$

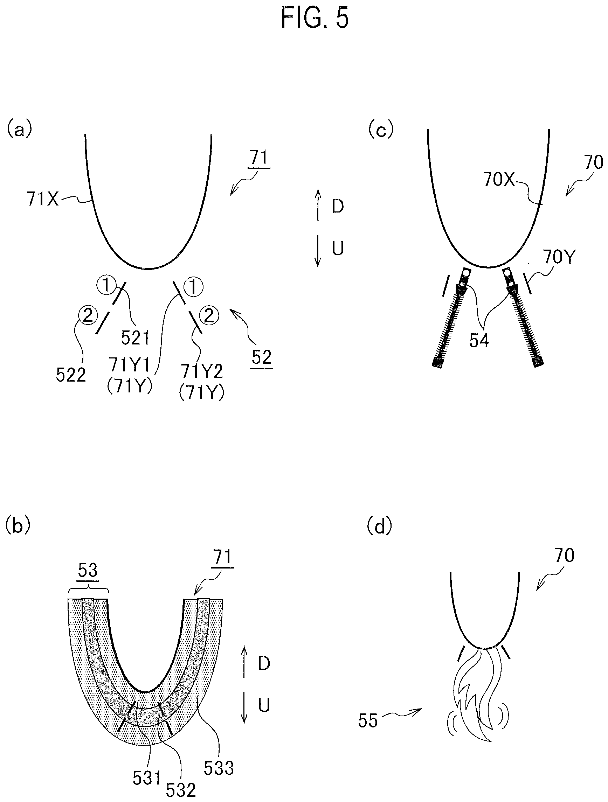

Next, a disposable diaper for pet of a modified example will be explained, referring to Fig. 5. In the following descriptions of the modified example, all structures similar to those in the first embodiment will be given same reference sings, so as to avoid redundant explanation. Note that in the following illustration of the modified example, only the tail hole 70 and the tail mark portion 51 on the disposable diaper for pet when viewed from the back side are shown for the convenience of explanation, leaving all other components not shown.

Fig. 5(a) illustrates a tail hole 71 and at least one tail mark portion 52 in the second embodiment. In the second embodiment, a plurality of cut parts 71Y for the tail hole 71 is arranged in the direction departing from a through-hole 71X, with a space in between. Each cut part 71Y has a first cut part 71Y1 and a second cut part 71Y2 positioned more distant from the through-hole than the first cut part 71Y1. The first cut parts 71Y1 and the second cut parts 71Y2 are arranged so as to extend radially in the direction departing from the through-hole 71X.

There is a plurality of tail mark portions 52, enabling pointing of stepwise changes of the through-hole, with the aid of the plurality of cut parts. Each tail mark portion 52 indicates that the size of the through-hole may be enlarged stepwise, with the aid of the cut parts. In the tail hole 71 in the second embodiment, the size of the through-hole 71X can be varied by a first step enlarging the through-hole with the aid of the first cut parts 71Y1, and a second step further enlarging the through-hole with the aid of the second cut parts 71Y2. The tail mark portion 52 has a first tail mark portion 521 given by numeral "1" that teaches manipulation of the first step with the aid of the first cut parts 71Y1, and a second tail mark portion 522 given by numeral "2" that teaches manipulation of the second step with the aid of the second cut parts 71Y2. The user can be aware of the presence of the plurality of cut parts, and can adjust the size of the through-hole to a degree suited for the individual pets.

The first tail mark portion 521 may point the first cut parts 71Y1, and the second tail mark portion 522 may point the second cut parts 71Y2. The user can be aware of the presence of the plurality of cut parts, and can adjust the size of the through-hole to a degree suited for the individual pets.

The first tail mark portion 521 may teach the order of manipulation of the first cut parts 71Y1, and the second tail mark portion 522 may teach the order of manipulation of the second cut parts 71Y2. The user can understand the order of manipulation of the first cut parts 71Y, and can enlarge stepwise the size of the through-hole by the manipulation, to thereby adjust the size of the through-hole to a degree suited for the individual pets.

Fig. 5(b) illustrates the tail hole 71 and a tail mark portion 53 in the third embodiment. The tail hole 71 in the third embodiment is same as the tail hole in the second embodiment. The tail mark portion 53 has a first tail mark portion 531, a second tail mark portion 532, and a third tail mark portion 533. Each of the first tail mark portion 531 to the third tail mark portion 533 has an arcuate form that extends conforming to a semicircle. The tail mark portion 53 has a first tail mark portion 531 arranged along the through-hole, a second tail mark portion 532 arranged on the side more distant from the through-hole 71X than the first tail mark portion 531, and a third tail mark portion 533 arranged on the side more distant from the through-hole 71X than the second tail mark portion 532. The first tail mark portion 531, the second tail mark portion 532, and the third tail mark portion 533 have different colors, indicating that the size of the through-hole 71X increases stepwise as the color changes.

Fig. 5(c) illustrates the tail hole 70 and a tail mark portion 54 in the fourth embodiment. The tail hole 70 in the fourth embodiment is same as the tail hole in the first embodiment. The tail mark portion 54 teaches the direction of manipulation of the cut parts 70Y. The user can understand the direction of manipulation of the cut parts 70Y, and can enlarge stepwise the size of the through-hole 70X by the manipulation, to thereby adjust the size of the through-hole to a degree suited for the individual pets. The tail mark portion 54 that teaches the direction of manipulation of the cut parts may have an illustration of zipper that teaches a direction along which the cut parts are torn, or may have an illustration of arrow that teaches a direction along which the cut parts are torn, or may have a text comment that teaches a direction along which the cut parts are torn.

Fig. 5(d) illustrates the tail hole 70 and a tail mark portion 55 in the fifth embodiment. The tail hole 70 in the fifth embodiment is same as the tail hole in the first embodiment. The tail mark portion 55 has a design portion representing a wearing target pet. More specifically, the tail mark portion of a disposable diaper for dog may have dog-related illustrations including a tail of dog, a body of dog, a face of dog, and a dog's favorite (bone, for example); and the tail mark portion of a disposable diaper for cat may have cat-related illustrations including a tail of cat, a body of cat, a face of cat, and a cat's favorite (fish or bristle grass, for example). The user can recognize the wearing target pet, with the aid of the tail mark portion.

Next, an exemplary method for manufacturing the thus designed disposable diaper for pet will be explained. The method for manufacturing the disposable diaper for pet may include:

a sheet feeding step of feeding a continuous sheet having sheet components arrayed therein; a marking step of giving a mark portion by printing on the continuous sheet to provide tail mark portion 51; and an absorbent core laminating step of laminating the absorbent core 30 on the continuous sheet having the tail mark portion 51.

The sheet feeding step, feeding the continuous sheet having sheet components arrayed therein, takes part in feeding of the continuous sheet in which sheet components, composing any one of the topsheet 10, backsheet and core wrap, are arrayed. The marking step takes part in giving the tail mark portion 51 on the continuous sheet. The tail mark portion 51 may be given by printing, or by embossing. The core laminating step takes part in detection of the tail mark portion 51 on the continuous sheet. The tail mark portion 51 may be detected by detecting a position thereof using an image capturing means such as camera. The core laminating step takes part in adjustment of a position of lamination of the absorbent core 30, referring to the position of the tail mark portion 51. Adjustment of the position of lamination of the absorbent core 30 means adjustment of the positional relation between the absorbent core 30 and the continuous sheet. More specifically, the positional relation between the continuous sheet and the absorbent core 30 is adjustable by controlling the rate of feeding of the continuous sheet. Alternatively, an adjustment mechanism may be arranged adjacent to the sides, in a cross direction orthogonal to the feeding direction, of the continuous sheet, and a position in the orthogonal direction of the continuous sheet may be adjusted using such adjustment mechanism. With such method for manufacturing, the positional relation between the tail mark portion 51 and the absorbent core 30 may be kept constant. Hence, the disposable diaper when placed referring to the tail mark portion 51 will have the absorbent core 30 whose placement position is prevented from being misaligned one by one. With suppression of shift of the tail mark portion 51 over the whole range of the disposable diaper, alignment referring to such tail mark portion 51 will be enabled with improved accuracy.

Next, a packaged article having a plurality of disposable diapers for pet enclosed therein will be explained. The packaged article includes a plurality of disposable diapers for pet, and a wrapper for enclosing the plurality of disposable diapers for pet. The plurality of disposable diapers for pet includes a first disposable diaper having the first decoration part printed on the backsheet, and a second disposable diaper having the second decoration part designed differently from the first decoration part printed on the backsheet. The first decoration part and the second decoration part may only have design different from each other, and may be different from the first decoration part and the second decoration part in the second embodiment. The disposable diaper in this embodiment has the first decoration part or the second decoration part, printed on the back film of the backsheet. With such first disposable diaper having the first decoration part and the tail mark portion, and the second disposable diaper having the second decoration part and the tail mark portion, this embodiment will have enhanced decorativeness, thereby can attract attention of the user, and can improve recognizability of the tail mark portion 51. The first decoration part and the second decoration part are parts having a design other the tail mark portion 51, and may have not only picture or pattern, but also background color solely represented by color.

The embodiments of the present invention have been described above in detail, it is apparent for persons who have ordinary skill in the art that the embodiments described in this specification do not limit the scope of the present invention. Changes and modifications may apply to the embodiments of the present invention without departing from the spirit and scope of the present invention that are defined by the description of the appended claims. The description of the present specification, therefore, has been understood to be illustrative and is in no way intended to limit the scope of the invention.

The entire contents of Japanese Patent Application No. 2019-094871 (filed on May 20, 2019) and the entire contents of Japanese Patent Application No. 2019-203979 (filed on November 11, 2019) have been incorporated herein by reference.

[INDUSTRIAL APPLICABILITY]

According to the present invention, it is possible to provide a disposable diaper for pet having the tail hole, which can be placed in the suitable position.

[REFERENCE SIGNS LIST]

1 : Disposable diaper for pet,
2 : Main body portion
10 : Topsheet

20 : Backsheet
30: absorbent core
32: core wrap
40: Binding portion
45: target part
51,52,53,54,55: Mark portions
521,531: first tail mark portion
522,532: second tail mark portion
533: third tail mark portion
70,71: tail hole
70X,71X: through-hole
70Y,71Y: cut part
71Y1: first cut part
71Y2: second cut part
90: fastening tape
W : Waistline direction
Z : Cross direction

**Claims**

1. A disposable diaper for pet, comprising:

   a waistline direction disposed along a waistline direction of a pet;
   a cross direction orthogonal to the waistline direction, and extends in a direction connecting a ventral side and a dorsal side of the pet;
   a main body portion having a topsheet, a backsheet, and an absorbent core arranged between the topsheet and the backsheet; and
   a tail hole through which a tail of pet can be inserted, and
   the disposable diaper for pet having a tail mark portion that indicates a position of the tail hole.

2. The disposable diaper for pet according to claim 1, wherein a minimum distance, in plan view of the disposable diaper for pet, between the tail mark portion and the tail hole is equal to or smaller than a maximum size of the tail hole.

3. The disposable diaper for pet according to claim 1 or 2, wherein at least a part of the tail mark portion, in plan view of the disposable diaper for pet, is arranged in a region that overlaps the tail hole.

4. The disposable diaper for pet according to any one of claims 1 to 3, wherein the tail mark portion indicates a direction of placement of the disposable diaper for pet.

5. The disposable diaper for pet according to any one of claims 1 to 4, wherein the tail hole has a through-hole through which the tail of pet can be inserted, and a cut part that can enlarge the through-hole, and the tail mark portion indicates a position of the cut part.

**6.** The disposable diaper for pet according to claim 5, wherein the cut part has a first cut part, and a second cut part positioned more distant from the through-hole than the first cut part, and
the tail mark portion has a first tail mark portion that indicates a position of the first cut part, and a second tail mark portion that indicates a position of the second cut part.

**7.** The disposable diaper for pet according to claim 6, wherein the first tail mark portion teaches order of manipulation of the first cut part, and
the second tail mark portion teaches order of manipulation of the second cut part.

**8.** The disposable diaper for pet according to any one of claims 5 to 7, wherein the tail mark portion teaches a direction of manipulation of the cut part.

**9.** The disposable diaper for pet according to any one of claims 1 to 8, wherein the backsheet of the disposable diaper for pet has the tail mark portion, and a decoration part designed differently from the tail mark portion printed on the backsheet.

**10.** The disposable diaper for pet according to any one of claims 1 to 9, wherein the tail mark portion has a design portion representing a wearing target pet.

**11.** A method for manufacturing a disposable diaper for pet, the method comprising:

a sheet feeding step of feeding a continuous sheet having sheet components arrayed in the continuous sheet;
a marking step of giving a tail mark portion on the continuous sheet; and
a core laminating step of laminating an absorbent core on the continuous sheet having the tail mark portion,
the core laminating step being designed to detect a position of the tail mark portion on the continuous sheet, and to adjust a position of lamination of the absorbent core referring to the position of the tail mark portion.

# FIG. 1

# FIG. 2

# FIG. 3

FIG. 4

# FIG. 5

(a)

71

71X

71Y1
(71Y)

71Y2
(71Y)

52

521

522

① ②

D

U

(c)

70

70X

70Y

54

(b)

53

71

531 532 533

D

U

(d)

70

55

Europäisches
Patentamt

European
Patent Office

Office européen
des brevets

# EUROPEAN SEARCH REPORT

Application Number

EP 20 17 5178

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 895 901 B1 (HOWARD SANDRA SMITH [US]) 24 May 2005 (2005-05-24) * column 4, line 24 - column 5, line 67; claims; figures * | 1-11 | INV. A61F13/514 A01K23/00 A61B46/00 |
| X | EP 2 932 834 A1 (UNI CHARM CORP [JP]) 21 October 2015 (2015-10-21) * paragraphs [0057] - [0157]; figures * | 1-11 | |
| X | JP 3 141793 U (SUZU M) 22 May 2008 (2008-05-22) * abstract * | 1-11 | |
| | | | TECHNICAL FIELDS SEARCHED (IPC) A61F A01K A61B |

The present search report has been drawn up for all claims

| Place of search | Date of completion of the search | Examiner |
|---|---|---|
| Munich | 29 September 2020 | Boccignone, Magda |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 17 5178

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2020

| Patent document cited in search report | | Publication date | Patent family member(s) | | Publication date |
|---|---|---|---|---|---|
| US 6895901 | B1 | 24-05-2005 | NONE | | |
| EP 2932834 | A1 | 21-10-2015 | CN | 104918481 A | 16-09-2015 |
| | | | EP | 2932834 A1 | 21-10-2015 |
| | | | JP | 5755631 B2 | 29-07-2015 |
| | | | JP | 2014117175 A | 30-06-2014 |
| | | | US | 2015305307 A1 | 29-10-2015 |
| | | | WO | 2014091849 A1 | 19-06-2014 |
| JP 3141793 | U | 22-05-2008 | NONE | | |

EPO FORM P0459

For more details about this annex : see Official Journal of the European Patent Office, No. 12/82

**EP 3 741 341 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003210062 A
- JP 2019094871 A
- JP 2019203979 A